# EUROPEAN PATENT SPECIFICATION

(11) **EP 3 991 699 B1**
(45) Date of publication and mention of the grant of the patent: **26.11.2025**
(21) Application number: 20832249.5
(22) Date of filing: 26.06.2020
(51) Int. Cl.: A61F 2/90, A61F 2/91, A61F 2/966, A61F 2/97, A61F 2/24, A61B 17/12

(54) **VASCULAR IMPLANT, DELIVERY DEVICE AND MEDICAL APPARATUS**
VASKULÄRES IMPLANTAT, ABGABEVORRICHTUNG UND MEDIZINISCHES GERÄT
IMPLANT VASCULAIRE, DISPOSITIF D'INTRODUCTION ET APPAREIL MÉDICAL

(30) Priority: 28.06.2019 CN 201910580250
(43) Date of publication of application: 04.05.2022
(73) Proprietor: MicroPort NeuroTech (Shanghai) Co., Ltd., Shanghai 201318 (CN)
(72) Inventor: ZHAO, Hanyi, Shanghai 201318 (CN); TIAN, Hao, Shanghai 201318 (CN); PENG, Qing, Shanghai 201318 (CN); WANG, Qinfen, Shanghai 201318 (CN)
(74) Representative: Patentship Patentanwaltsgesellschaft mbH
(86) International application number: PCT/CN2020/098312
(87) International publication number: WO 2020/259640

(56) References cited:
- CN-A- 108 378 960
- CN-A- 109 381 286
- CN-A- 110 151 368
- CN-U- 208 582 554
- CN-U- 210 749 680
- US-A1- 2004 078 071
- US-A1- 2004 204 749
- US-A1- 2006 089 703
- US-A1- 2007 293 930
- US-A1- 2009 306 761
- US-A1- 2014 142 688

## Description

### TECHNICAL FIELD

The present invention relates to the technical field of medical instruments and, more specifically, to a vascular implant, a delivery device and a medical apparatus.

### BACKGROUND

As a therapy for treating vascular aneurysms, minimally invasive intervention typically involves delivering a vascular implant, for example, but not limited to, a stent, an embolization coil or an aneurysm occluder, to a lesion site using a delivery device. The delivery device usually includes a delivery shaft, which is brought into engagement, and received together within a delivery sheath, with the vascular implant, so as to be able to deliver the vascular implant within the delivery sheath to the site and release it there.

Currently, the engagement of the vascular implant with the delivery shaft that enables its delivery in the delivery sheath is usually accomplished by static friction. That is, the vascular implant is contracted and sleeved over the delivery shaft so that they move together in the delivery sheath as a whole, without any mechanical connection between them. However, in practice, it is difficult to control the magnitude of the friction, and thus it is likely for the vascular implant to dislodge (i.e., fall off the delivery shaft). When this happens, stable delivery of the vascular implant is impossible, adversely affecting the surgical outcomes.

US 2009/306761 A1 discloses a prosthesis delivery system. US 2004/078071 A1 discloses an expandable stent and delivery system therefor. US 2004/204749 A1 discloses a stent delivery system.

### SUMMARY OF THE INVENTION

The invention is set out in the appended set of claims.

To this end, it is an objective of the present invention to provide a vascular implant, a delivery device and a medical apparatus, in which the vascular implant can be kept stationary axially with respect to a delivery shaft and thus delivered in a more stable manner without dislodgement.

To achieve the above objective, the present invention provides a vascular implant, for delivery to a target site by a delivery device comprising a delivery shaft and a chamber, wherein the vascular implant comprises a proximal end defining a first engagement structure configured for detachable retaining engagement with the delivery shaft, the first engagement structure and the delivery shaft being able to be received in or removed from the chamber,
wherein when the first engagement structure and the delivery shaft are received in the chamber, the first engagement structure is confined by the chamber and remains in retaining engagement with the delivery shaft, so that the vascular implant is axially locked to the delivery shaft, and
wherein when the first engagement structure is removed from the chamber, the first engagement structure is no longer confined by the chamber and is detachable from the delivery shaft, so that the vascular implant is axially unlocked from the delivery shaft.

According to the present invention, the first engagement structure comprises at least one first engagement member configured to remain in retaining engagement with the delivery shaft when confined by the chamber and to become detachable from the delivery shaft when no longer confined by the chamber, wherein the first engagement member defining a gap configured to:
disallow passage of the delivery shaft therethrough when the first engagement structure and the delivery shaft are received in the chamber to maintain the first engagement member in retaining engagement with the delivery shaft; and
allow passage of the delivery shaft therethrough when the first engagement structure is removed from the chamber to make the first engagement member detachable from the delivery shaft.

According to the invention, the first engagement member comprises two or more retention lugs which are spaced apart from one another along a circumferential direction of the vascular implant and define the gap therebetween.

According to the invention, the retention lugs are configured to be deformable along an axial direction of the vascular implant so that the first engagement member is deformable along the axial direction of the vascular implant.

According to a preferred embodiment of the invention, at least one of the two or more retention lugs is made of a radiopaque metallic material.

According to aspects of the disclosure, the first engagement structure comprises two or more said first engagement members which are spaced from one another in an axial direction of the vascular implant.

According to aspects of the disclosure, the retention lugs in the two or more first engagement members are aligned with one another in the axial direction of the vascular implant.

According to aspects of the disclosure, the first engagement structure comprises two or more first engagement member groups spaced from one another in an axial direction of the vascular implant,
each of the first engagement member groups comprising two or more said first engagement members which are spaced from one another in an axial direction of the vascular implant, and
wherein the retention lugs in any one of the first engagement member groups are aligned with one another in the axial direction of the vascular implant and are staggered from the retention lugs in any other one of the first engagement member groups in the axial direction of the vascular implant.

According to a preferred embodiment of the invention, the vascular implant is a self-expanding implant.

According to a preferred embodiment of the invention, the vascular implant is a stent, an embolization coil or an aneurysm occluder.

According to configurations not covered by the present application, a delivery device is provided, for delivering a vascular implant to a target site, the delivery means comprising:
a delivery shaft comprising a second engagement structure configured for detachable retaining engagement with a proximal end of the vascular implant; and
a chamber configured to receive therein the delivery shaft and the proximal end of the vascular implant,
wherein when the proximal end of the vascular implant and the delivery shaft are received in the chamber, the proximal end of the vascular implant is confined by the chamber and remains in retaining engagement with the second engagement structure, so that the vascular implant is axially locked to the delivery shaft, and
wherein when the proximal end of the vascular implant is removed from the chamber, the proximal end of the vascular implant is no longer confined by the chamber and is thus detachable from the second engagement structure, so that the vascular implant is axially unlocked from the delivery shaft.

According to aspects of the disclosure, the second engagement structure comprises at least one second engagement member configured for detachable retaining engagement with the proximal end of the vascular implant, and
wherein the delivery shaft further comprises a shaft body provided thereon with at least one projection serving as the second engagement member, so that the proximal end of the vascular implant is allowed to be retained on both sides of the at least one projection or between at least two said projections.

According to aspects of the disclosure, the second engagement structure comprises at least one second engagement member configured for detachable retaining engagement with the proximal end of the vascular implant, and
wherein the delivery shaft further comprises a shaft body provided therein with at least one recess serving as the second engagement member, so that the proximal end of the vascular implant is allowed to be retained in the recess.

According to aspects of the disclosure, the second engagement member comprises a surface indent axially extending therethrough, the surface indent configured to receive therein part of the vascular implant.

According to aspects of the disclosure, two or more said surface indent are provided and arranged on a circumference of the second engagement member.

According to aspects of the disclosure, the proximal end of the vascular implant comprises at least one first engagement member configured for detachable retaining engagement with the second engagement member, the first engagement member comprising two or more retention lugs that are spaced from one another along a circumferential direction of the vascular implant so that a gap is formed between the two or more retention lugs, and
wherein when the proximal end of the vascular implant and the delivery shaft are in retaining engagement with each other and received in the chamber, the surface indent is configured to disallow passage of any of the retention lugs therethrough.

According to aspects of the disclosure, the second engagement structure is made of a radiopaque metallic material.

According to aspects of the disclosure, the delivery device further comprises a confinement member which comprises the chamber and is disposed over the delivery shaft.

According to aspects of the disclosure, the delivery device further comprises a delivery sheath configured for loading the vascular implant therein, wherein the delivery shaft is configured to be movably inserted in the delivery sheath, and wherein the delivery sheath serves as the confinement member.

According to aspects of the disclosure, the delivery device further comprises a delivery sheath configured for loading the vascular implant therein, wherein the delivery shaft is configured to be movably inserted in the delivery sheath, and wherein the confinement member is configured to be movably inserted in the delivery sheath and sleeved over both the proximal end of the vascular implant and the delivery shaft.

According to configurations not covered by the present application, a medical apparatus is also provided, comprising:
the above vascular implant; and
the above delivery device,
wherein the first engagement structure of the vascular implant is configured for detachable retaining engagement with the second engagement structure of the delivery shaft.

The vascular implant, delivery device and medical apparatus provided in the present invention have the following advantages:
First, the detachable retaining engagement of the proximal end of the vascular implant with the delivery shaft in the delivery device maintains the vascular implant, when being delivered, axially stationary relative to the delivery shaft. This avoids dislodgement of the vascular implant during its delivery, making the delivery more stable and thus resulting in reduced surgical complexity, enhanced surgical safety and improved treatment outcomes.

Second, in the medical apparatus, with the aid of the chamber in the delivery device, desirable axial locking of the vascular implant to the delivery shaft and its axial unlocking therefrom can be easily achieved, resulting in reduced surgical complexity, increased surgical efficiency and enhanced engagement reliability.

Third, the proximal end of the vascular implant preferably includes two or more first engagement member groups, which are spaced from one another in the axial direction of the vascular implant and the retention lugs in different first engagement member groups are staggered in the axial direction of the vascular implant. In this way, the retention lugs can be confined on different circumferences that are spaced apart axially, resulting in a reduced overall size of the vascular implant and the delivery device and allowing the vascular implant to be delivered by an even smaller delivery device.

Fourth, the delivery device preferably includes the confinement member, which provides the chamber and is preferably to be movably inserted in the delivery sheath so as to confine the first engagement structure defined at the proximal end of the vascular implant. This enables entire release and entire withdrawal of the vascular implant. For example, after the vascular implant and the delivery shaft are pushed out of the delivery sheath at the target site, the vascular implant remains engaged with the delivery shaft. At this point, the vascular implant can be withdrawn and released again at a later time when it is determined that it would be appropriate to do so. Relative movement between the confinement member and the delivery shaft can release the confinement of the confinement member and thus allows disengagement and separation of the vascular implant from the delivery shaft. In this way, the vascular implant can be released more accurately, resulting in improved treatment outcomes.

### BRIEF DESCRIPTION OF THE DRAWINGS

Fig. 1 is a schematic diagram showing the structure of a self-expanding braided stent according to an embodiment of the present invention;
Fig. 2a is a schematic diagram showing the structure of a retention lug formed at a proximal end of the self-expanding braided stent where the filaments are welded together according to an embodiment of the present invention;
Fig. 2b is a zoom-out schematic view of retention lugs at the proximal end of the self-expanding braided stent according to an embodiment of the present invention;
Figs. 3a to 3d are schematic diagrams showing the structures of variants of a second engagement member according to embodiments of the present invention;
Fig. 4 is a schematic diagram showing the structure of a delivery shaft including two second engagement members according to Embodiment 1 of the present invention;
Fig. 5 is a schematic diagram showing the engagement between the self-expanding braided stent and a delivery device according to Embodiment 1 of the present invention;
Fig. 6a schematically illustrates how a gap is formed in a first engagement member according to Embodiment 1 of the present invention;
Fig. 6b is a schematic diagram showing that the second engagement member has a maximum diameter that is greater than a maximum span of the gap in the first engagement member according to Embodiment 1 of the present invention;
Fig. 7 is an enlarged view of a retention lugs at the proximal end of the self-expanding braided stent according to the present disclosure;
Fig. 8 is a schematic diagram showing the structure of the delivery shaft including one second engagement member according to the present disclosure;
Fig. 9 schematically illustrates how retaining engagement is established between the first and second engagement members according to the present disclosure;
Fig. 10 is a schematic diagram showing that the delivery device and the self-expanding braided stent are engaged with each other according to the present disclosure, where the proximal end of the self-expanding braided stent does not disengage from a delivery sheath yet;
Fig. 11 is an enlarged view of retention lugs at the proximal end of the self-expanding braided stent according to the present disclosure;
Fig. 12 is a schematic diagram showing that the delivery device and the self-expanding braided stent are engaged with each other according to the present disclosure, where the proximal end of the self-expanding braided stent does not disengage from the delivery sheath yet;
Fig. 13 is an enlarged view of Fig. 12, which shows the self-expanding braided stent and the delivery shaft are in retaining engagement with each other; and
Fig. 14 is a schematic diagram showing that the delivery device and the self-expanding braided stent are engaged with each other according to the present disclosure, where the proximal end of the self-expanding braided stent does not disengage from a release tube yet.

In these figures, 1 denotes the stent; 11, the proximal end; 12, a distal end; 13, a retention lug; 14 and 15, first engagement member groups; 2, the first engagement member; 3, the second engagement member; 4, the delivery shaft; 5, the delivery sheath; 6, the release tube;
G, surface indents; S1, S2 and S, filaments; and Q, welded joints.

Like reference numerals across the several views refer to identical or equivalent features.

### DETAILED DESCRIPTION

Objects, advantages and features of the present invention will become more apparent from the following more detailed description thereof made in conjunction with the accompanying drawings. Note that the figures are provided in a very simplified form not necessarily drawn to exact scale for the only purpose of helping to explain the disclosed embodiments in a more convenient and clearer way.

As used herein and in the appended claims, the singular forms "a", "an" and "the" include plural referents, unless the context clearly dictates otherwise. As used herein and in the appended claims, the term "or" is generally employed in the sense of "and/or", "plurality" of "two or more", and "several" of "one or more", unless the context clearly dictates otherwise.

In the following description, the terms "distal" and "proximal" may be used for the sake of ease of description. When used to describe a medical instrument, the term "proximal" refers to an end thereof close to an operator who is operating the instrument, while the term "distal" refers to an end away from the operator. The following description sets forth numerous specific details in order to provide a more thorough understanding of the present invention. However, it will be apparent to those skilled in the art that the present invention can be practiced without one or more of these specific details. In other instances, well-known technical features have not been described in order to avoid unnecessary obscuring of the present invention.

The core concept of the present disclosure is to provide a medical apparatus including a vascular implant and a delivery device for delivering the vascular implant to a target site, the delivery device including a delivery shaft and a chamber.

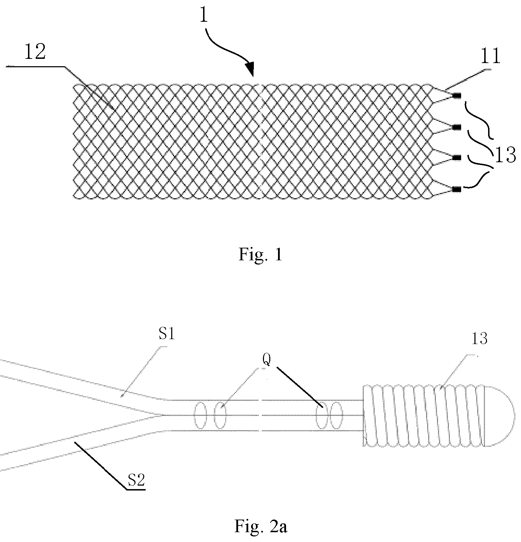

The invention relates to a vascular implant according to Figs. 1-6. The vascular implant of the present invention has a proximal end defining a first engagement structure, and the delivery shaft in the delivery device of the present invention defines a second engagement structure. The first engagement structure is configured for detachable retaining engagement with the second engagement structure so that the vascular implant remains axially stationary relative to the delivery shaft.

The first engagement structure is configured to be received together with the delivery shaft within the chamber so that it is confined by the chamber and remains in engagement with the second engagement structure. As a result, the vascular implant is axially locked to the delivery shaft. On the contrary, when the first engagement structure is released from, and thus not confined anymore by, the chamber, it is detachable from the second engagement structure, axially unlocking the vascular implant from the delivery shaft. Such detachable mechanical engagement established between the vascular implant and the delivery shaft prevents the vascular implant from dislodgement while it is being delivered, enabling stable delivery of the vascular implant, which results in easier surgical operation, reduced surgical complexity, enhanced surgical safety and improved treatment outcomes.

The delivery device further includes a confinement member that defines the chamber. The confinement member is configured to be disposed over the delivery shaft. In one embodiment, the delivery device further includes a delivery sheath in which the vascular implant can be loaded. Moreover, the delivery shaft is movably inserted within the delivery sheath. The delivery sheath may serve as the confinement member. In an alternative embodiment, the confinement member may be a release tube, which is movably inserted in the delivery sheath so as to sleeved over the delivery shaft and surround both the first engagement structure and the delivery shaft. The first engagement structure may include one, two or more first engagement members for detachable retaining engagement with the second engagement structure. Correspondingly, the second engagement structure may include one, two or more second engagement members for detachable retaining engagement with the first engagement structure.

According to the present invention, the retaining engagement may be accomplished mainly by:
(1) retaining at least one first engagement member between at least two second engagement members;
(2) retaining at least one second engagement member between at least two first engagement members; and/or
(3) retaining at least one first engagement member in at least one second engagement member.

In practice, retaining engagement may be accomplished by one or combinations of the above three ways. Moreover, it is not limited to being accomplished by a single retention point, and two or more retention points may be alternatively created to result in firmer engagement.

According to the present invention, the vascular implant is typically an implant for treating a vascular aneurysm, such as a stent, an embolization coil or an aneurysm occluder. Alternatively, it may also be used in vasodilation treatment, blood clot capturing or the treatment of other diseased lumens. For example, when the confinement member is implemented as the delivery sheath, vascular implant of the present invention may be delivered in a manner described below.

First of all, the vascular implant is loaded by sleeving it over the delivery shaft and then inserting them together into the delivery sheath so that the first and second engagement structures remain engaged with each other under the action of radial confinement by the delivery sheath. As a result, the vascular implant is axially locked to the delivery shaft.

Subsequently, the vascular implant is pushed forward (i.e., delivered) by the delivery shaft so that it entirely (including the proximal end thereof) moves out of the delivery sheath. As a result, no longer radially confined by the delivery sheath, the vascular implant extends (or expands), causing disengagement between the first and second engagement structures and thus allowing release of the vascular implant.

The expansion of the vascular implant subsequent to its movement out of the delivery sheath may be either self-expansion or passive expansion. The term "self-expansion" means that the vascular implant possesses self-expanding properties, which allow it to expand or extend by itself when in absence of external constraints against such expansion. The term "passive expansion" means that the vascular implant has to be expanded or extend by an external mechanism such as a balloon when in absence of external constrains against such expansion.

In an alternative embodiment, the confinement member is a release tube, which is movably inserted in the delivery sheath so as to sleeve over the delivery shaft. In practical operation, the release tube may also be used for entire release and entire retrieval of the vascular implant, as will be described in detail below. This makes it easy to observe the position and condition of the released implant and to adjust the position thereof if necessary, allowing accurate release of the vascular implant and thus improved treatment outcomes.

The vascular implant and delivery device are further described below by way of several examples with reference to the accompanying drawings. Although the following description is made in the context of the vascular implant being a stent, modifications may be made thereto by those skilled in the art so that it is applicable to another implant than a stent.

As shown in Fig. 1, the vascular implant may be a stent 1. According to configurations not covered by the present application, the stent 1 may have a cut structure, or a combination of a cut structure and a braided structure. According to the present invention, the stent 1 has a braided structure. Moreover, the stent 1 may be either self-expanding or passively expandable. The cut structure may be fabricated from a shape memory material or an elastic material, including, but not limited to, nickel-titanium alloys. The braided structure may be formed by braiding filaments of a metallic or macromolecular material, including, but not limited to, nickel-titanium alloys, stainless steel and macromolecular polymers.

Although the following description is made mainly in the context of the stent being a self-expanding braided structure, modifications may be made thereto by those skilled in the art so that it is applicable to another stent than a self-expanding braided one.

With continued reference to Fig. 1, the stent 1 has a proximal end 11 and an opposite distal end 12. The proximal end of the stent 1 may be formed by a single filament or binding two or more filaments, or be formed by braiding a single filament, which is not limited in the present invention. Exemplary means for binding the filament segments together may include, but are not limited to, twisting, bonding and welding. In a non-limiting example shown in Fig. 2a, two adjacent filaments S1 and S2 at the proximal end 11 may be welded together at a plurality points Q.

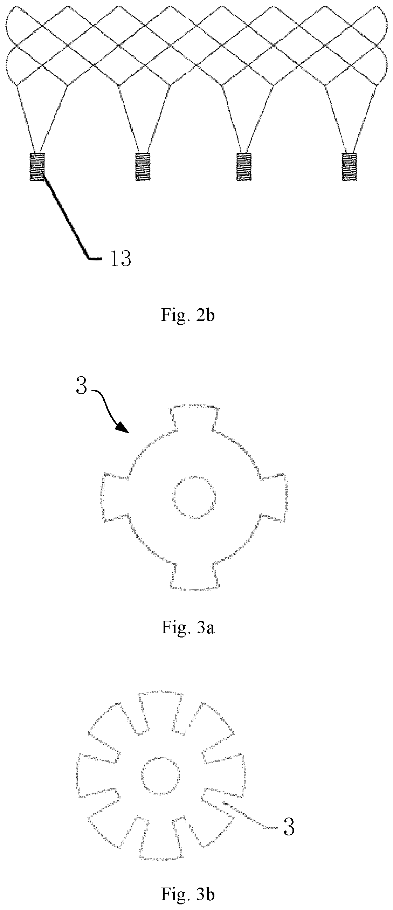

Referring to Fig. 2b, in conjunction with Fig. 2a, either of the filaments S1 and S2 and/or both of the filaments S1 and S2 may be further wound to form a retention lug 13 in the shape of a coiled tube. According to the present invention, a retention lug 13 is provided at the proximal end 11 of the stent 1 and formed of a separate filament that is wound into a coiled tube. Still alternatively, a separate retention lug 13 in the shape of a coiled tube is sleeved over the filaments bound together at the proximal end 11. The present invention is not limited to any particular formation of the retention lug 13 at the proximal end of the stent 1.

In this embodiment, the retention lug 13 is a hollow tubular structure in the shape of a coiled tube. In alternative embodiments, the retention lug 13 may be a solid structure, such as a solid cylinder connected to proximal end of the two bound filaments. Therefore, the retention lug 13 is not limited to having any particular structure, as long as it is easy to attach to the filaments. According to the present invention, the retention lug 13 is tubular and surrounds the filaments and is coiled to have some elasticity. When shaped as a coiled tube, the retention lug 13 can be compressed in the axial direction of the stent 1 into a configuration that allows easier engagement. The retention lug 13 may be formed of a macromolecular or metallic material, including, but not limited to, stainless steel and nickel-titanium alloys. Preferably, the retention lug 13 is made of a radiopaque metallic material, which makes it easy to check the position of the proximal end 11 of the stent 1 during surgical operation.

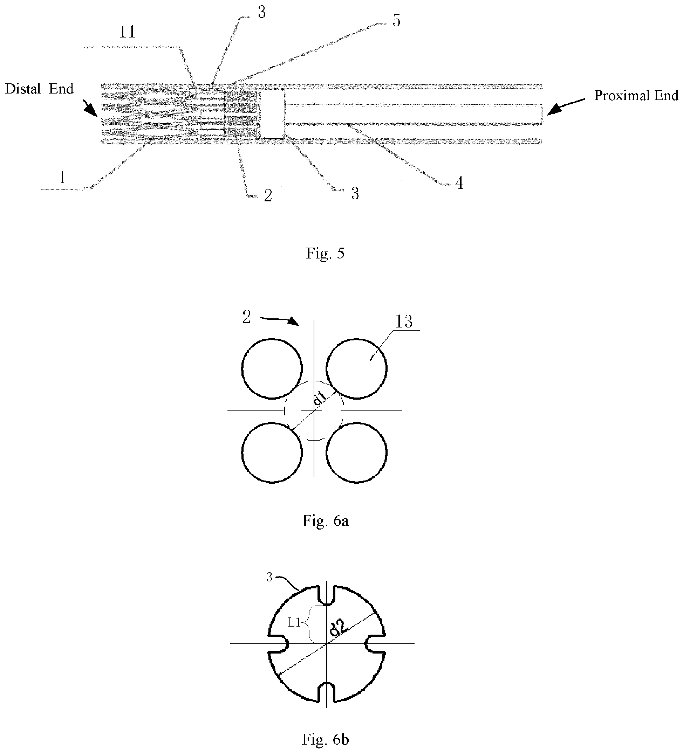

In embodiments of the present invention, the proximal end 11 of the stent 1 defines a first engagement structure including at least one first engagement member 2 (see Fig. 6a), which is configured to engage with the delivery shaft when confined in the chamber and become detachable from the delivery shaft upon being released from the confinement of the chamber. Preferably, the first engagement member 2 include two or more retention lugs 13, which are arranged in symmetry with respect to the axis of the stent 1 (i.e., circumferentially symmetrically or centrosymmetrically with respect to the stent 1) so that there is a gap (indicated at d1 in Fig. 6a) left between the retention lugs 13. The gap may vary in size principally as a result of compression and expansion of the stent. For example, in case of the confinement member being implemented as the delivery sheath, before being loaded into the delivery sheath, the stent 1 is not confined or compressed radially by the chamber yet, so the gap has a natural size that is large enough to allow a second engagement member 3 of the delivery shaft to pass therethrough so that the stent 1 can be loaded on the delivery shaft. When loaded in the delivery sheath in a state being engaged with (or retained on) the delivery shaft, the stent 1 is confined and compressed by the chamber, leading to shrinkage of the gap to a size that does not allow passage of the second engagement member 3 of the delivery shaft. That is, at this point, a maximum span (e.g., diameter) of the gap is smaller than a corresponding dimension (e.g., diameter) of the second engagement member 3, ensuring that the first engagement member 2 at the proximal end of the stent remains in retaining engagement with the second engagement member 3 that is arranged at a distal end of the delivery shaft. As a result, the stent 1 is axially locked to the delivery shaft, allowing delivery of the stent 1 within the delivery sheath using the delivery shaft. When released from the confinement of the chamber, the stent 1 will expand, allowing detachment of the first engagement member 2 from the second engagement member 3, i.e., axial unlocking of the stent 1 from the delivery shaft.

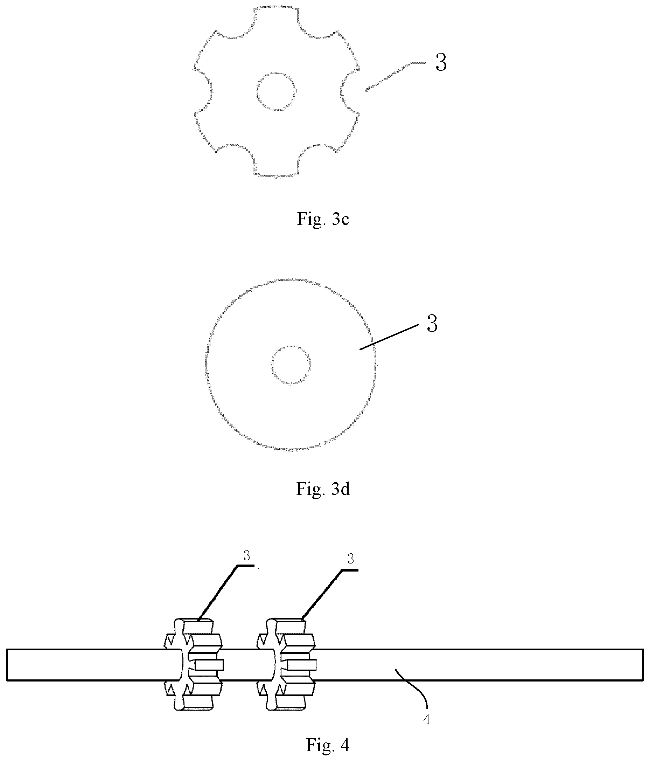

Reference is additionally made to Figs. 3a to 3d, in conjunction with Fig. 4, the delivery device includes the delivery shaft 4 that defines a second engagement structure including at least one second engagement member 3. In this embodiment, the delivery shaft 4 further includes a shaft body on which the at least one second engagement member 3 is disposed. In some embodiments, the at least one projection is formed on the shaft body, and the at least one projection forms the second engagement member 3. In such cases, at least one first engagement member 2 may be retained between at least two projections, or at least two second engagement members 3 may be retained on opposing ends of at least one projection. In some embodiments, the at least one recess is formed on the shaft body, and the at least one recess forms the second engagement member 3. In such cases, at least one first engagement member 2 may be retained in the at least one recess.

Although the following description is made in the context of the second engagement member 3 being implemented as a projection as an example of allowing easily achievable retaining engagement between the first and second engagement structures, modifications may be made thereto by those skilled in the art so that it is applicable to the cases where the second engagement member 3 is not a projection.

Combined reference is made to Fig. 2b, in practical use, the stent 1 is radially confined by the delivery sheath so that retention lugs 13 are engaged with the second engagement member 3 while being constrained by the delivery sheath and thus cannot escape. Such retaining engagement allows more stable delivery, and upon the retention lugs 13 being pushed out of the delivery sheath, the retention lugs 13 are no longer confined by the delivery sheath and can disengage from the second engagement member 3. Such disengagement can be established based on a simple structure, and is easy to achieve and allows quick release of the stent 1.

Compared to the prior art, the stent 1 is delivered in the delivery sheath while being mechanically retained on the delivery shaft. This substantially prevents dislodgement of the stent 1 during delivery and allows more stable delivery thereof. In addition, the retention structure is simple and allows easy locking and unlocking.

With continued reference to Figs. 3a to 3c, in some embodiments, a surface indent may be formed in the second engagement member 3, which extend axially therethrough. The surface indent may be in the form of a notch as shown in Fig. 3b or 3c, or may be defined by adjacent protrusions shown in Fig. 3a. As shown in Fig. 3d, in some alternative embodiments, the second engagement member 3 may be a cylinder that has a smooth surface and a cross-section shape, which may be, but is not limited to, circular, square, rectangular or profiled. The second engagement member 3 may be either solid or tubular, and in the latter case, it may be a coiled tube. The second engagement member 3 may be formed of a macromolecular or metallic material, including, but not limited to, stainless steel and nickel-titanium alloys. Preferably, the second engagement member 3 is made of a radiopaque metal material, which makes it easy to check the position thereof, in particular relative to the axis of the delivery sheath during the implantation procedure, thus determining the progress of release of the stent 1.

Two or more surface indents may be provided and disposed about an axis of the second engagement member 3 (i.e., spaced apart along a circumferential direction of the second engagement member 3, more preferably, in a symmetrical way). The proximal end 11 of the stent 1 may be so engaged that the filaments thereof are partially received in the surface indents of the second engagement member 3. This can, in an aspect, result in a reduced diameter of the compressed stent 1 and thus allow the use of a delivery sheath with a smaller diameter, and in another aspect, can strengthen the confinement of the stent 1 and the delivery shaft and thus additionally enhance the delivery stability of the stent.

Further, the proximal end 11 of the stent 1 may include several mesh cells, and the retention lugs 13 may be arranged at extremities of at least some of the mesh cells. That is, it is not necessary that each of the mesh cells is provided at its proximal extremity with a retention lug 13. Additionally, each first engagement member 2 includes two or more retention lugs 13, which are arranged on respective mesh cells so as to be in symmetry on the same circumference.

Retaining engagement established between the first and second engagement members 2 and 3 will be further described below, but the following description is in no way intended to limit the present invention. Rather, any and all modifications made in light of the following description are intended to be also embraced in the scope of the present invention.

### Embodiment 1

As shown in Fig. 4, in this embodiment, the delivery shaft 4 includes a second engagement structure that includes at least two second engagement members 3, which are spaced apart along the axial direction of the shaft body. For example, two second engagement members 3 are included, which are axially spaced from each other by a distance that allows at least one first engagement member 2 to be retained between the two second engagement members. When the first engagement member 2 is an elastic member that can be compressed between the two second engagement members 3, the axial distance between the two second engagement members 3 may be smaller than or equal to an axial length of the first engagement member 2 (when it is not compressed). Alternatively, according to a configuration not covered by the invention, the first engagement member 2 may be a rigid member without elasticity and deformability. In this case, the axial distance between the two second engagement members 3 may be greater than or equal to the axial length of the first engagement member 2. Moreover, in order to avoid significant axial displacement, there should not be an excessively large clearance left between any of the second engagement members 3 and the first engagement member 2. Further, the second engagement members 3 may also be elastic members so that they can be deformed to allow a first engagement member 2 to be compressed therebetween. In this case, the axial distance between the two second engagement members 3 may also be smaller than or equal to the axial length of the first engagement member 2.

As shown in Fig. 5, in conjunction with Fig. 2b, the first engagement member 2 includes four retention lugs 13, which are spaced apart on the same circumference and each arranged over filaments at the proximal end 11 of the stent 1. These retention lugs 13 may have the same or different structures, and may be aligned with one another on both sides or not. The delivery device further includes a delivery sheath 5. In practical operation, as shown in Fig. 5, the stent 1 is entirely compressed within the delivery sheath 5 so that the four retention lugs 13 at the proximal end 11 are confined and retained between the two second engagement members situated respectively at proximal and distal ends of the first engagement member 2. In this way, the stent 1 is prevented from moving in opposite directions.

In addition, indents may be formed in an outer surface of the distal one of the second engagement members 3, in which filaments at the proximal end 11 of the stent 1 may be received. Therefore, the two second engagement members 3 may have the same or different structures. Preferably, the distal second engagement member 3 is provided with surface indents, while the other is not.

As shown in Figs. 6a and 6b, when the stent 1 is (fully) compressed in the delivery sheath 5, a gap (indicated at d1 in the figure) is formed among the four retention lugs 13. When the gap has a regular shape, e.g., circular, the second engagement members 3 both have a diameter (e.g., a maximum span) d2 that is greater than a diameter d1 of the gap. When the gap has an irregular shape, the diameter d2 of the second engagement members 3 is greater than a maximum span of the gap. This ensures that the second engagement member 3 will not pass through the gap when the stent is being pushed.

With continued reference to Figs. 6a and 6b, when the stent 1 is fully compressed, a radial distance L1 for the surface indents may be equal to or greater than a radial distance (d1/2) for the gap. Alternatively, the radial distance L1 for the surface indents may also be smaller than the radial distance (d1/2) for the gap. That is, the surface indents are not limited to having any particular depth. However, in order to avoid any retention lug 13 from escaping through any surface indent, the surface indents are preferably sized so as to disallow passage of the retention lugs 13 therethrough. Preferably, a maximum span of each surface indent along the circumferential direction of the second engagement member 3 is designed to be smaller than a minimum span of each retention lug 13 along the circumferential direction of the stent 1.

In this way, in practical operation, as shown in Fig. 5, when the delivery shaft 4 is withdrawn (i.e., caused to move proximally), the first engagement member 2 is blocked by the distal second engagement member 3 and cannot move relative to the delivery shaft 4. On the contrary, when the delivery shaft 4 is pushed forward (i.e., caused to move distally), the first engagement member 2 is blocked by the proximal second engagement member 3 and also cannot move relative to the delivery shaft 4.

Configurations not covered by the present application are described in the following Embodiments 2-4.

### Embodiment 2

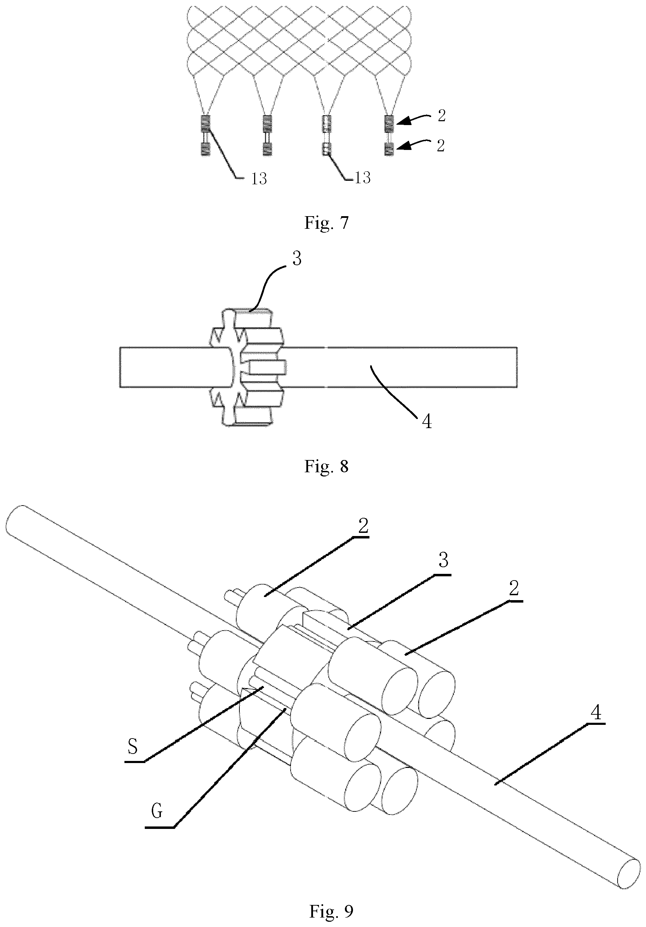

As shown in Figs. 7 and 8, in this embodiment, the second engagement structure of the delivery shaft 4 includes at least one second engagement member 3, while the first engagement structure at the proximal end 11 of the stent 1 includes at least two first engagement members 2, which are spaced from each other along the axial direction of the stent 1. For example, two first engagement members 2 are included, which are spaced by an axial distance allowing the at least one second engagement member 3 to be retained therebetween. The axial distance between the two first engagement members 2 may be greater or smaller than, or equal to an axial length of the second engagement member 3. For example, the first engagement members 2 may be elastic members that can be stretched or compressed.

As shown in Fig. 9, in practical operation, within the delivery sheath 5, the second engagement member 3 is retained between the two first engagement members 2, with filaments S between the two first engagement members 2 being received in surface indents G formed in the second engagement member 3. Moreover, during delivery, when the stent 1 is advanced (i.e., caused to move distally), the proximal one of the first engagement members 2 is blocked by the second engagement member 3, ensuring that there is no relative movement between the stent 1 and the delivery shaft 4. On the contrary, when the stent 1 is withdrawn, the distal one of the first engagement members 2 is blocked by the second engagement member 3. In this way, the stent 1 will not move relative to the delivery shaft 4 regardless of whether it is pushed forward or withdrawn, enabling stable delivery of the stent 1.

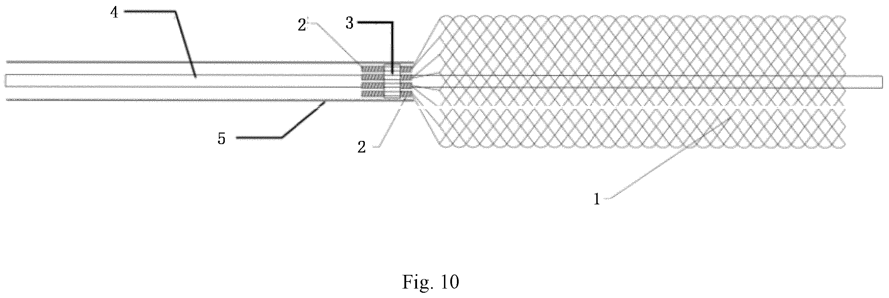

Further, as shown in Fig. 10, release of the stent 1 can be achieved simply by advancing it until the proximal end 11 thereof is entirely pushed out of the delivery sheath 5. Subsequently, the first engagement member 2 will flare or be caused to flare radially outward, disengaging and allowing release of the stent 1. Thus, as can be appreciated, the disengagement and release of the stent 1 is easy to achieve.

### Embodiment 3

In this embodiment, the first engagement structure at the proximal end of the stent includes two or more first engagement member groups, which are spaced apart from one another along the axial direction of the stent 1 and each includes two or more first engagement members 2. In each first engagement member group, the two or more first engagement members 2 are also spaced apart from one another along the axial direction of the stent 1, and all retention lugs 13 in the first engagement member are axially aligned with respect to the stent 1. Here, the term "axial aligned" means that when projected to a single plane perpendicular to the axis of the stent, the axial projections of all the retention lugs 13 in each first engagement member group coincide with one another.

In addition, the inventors have found that a greater radial size of the first engagement members 2 or a larger number of retention lugs 13 tends to lead to a larger overall size of the compressed stent 1 and the delivery device. In order to overcome this, retention lugs 13 in different first engagement member groups may be staggered along the axial direction of the stent 1 so that the retention lugs 13 in each first engagement member group can be confined on a separate circumference, resulting in a reduction in the size of the compressed stent and allowing the use of a smaller delivery sheath 5 in which the stent can be delivered.

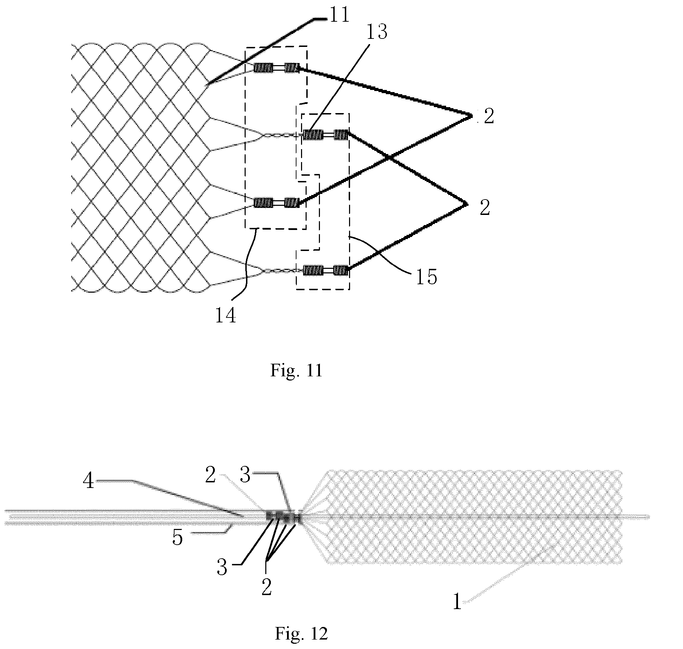

As shown in Fig. 11, in this embodiment, there are two first engagement member groups 14 and 15, as indicated by the dashed lines for clearer illustration. The first engagement member groups 14 and 15 are spaced apart from each other along the axial direction of the stent 1, and retention lugs 13 therein are axially staggered from each other. Here, the term "axially staggered" means that when projected to a single plane perpendicular to the axis of the stent, axial projections of the retention lugs 13 in the first engagement member group 14 do not coincide with those of the retention lugs 13 in the first engagement member group 15.

More specifically, the first engagement member group 14 includes two first engagement members 2, which are spaced from each other in the axial direction of the stent 1 so that one of them is closer to the distal end and the other to the proximal end. Moreover, the retention lugs 13 of the two first engagement members 2 in the first engagement member group 14 are axially aligned. Similarly, the first engagement member group 15 includes two first engagement members 2, which are spaced from each other in the axial direction of the stent 1 so that one of them is closer to the distal end and the other to the proximal end. Moreover, the retention lugs 13 of the two first engagement members 2 in the first engagement member group 15 are axially aligned.

For example, each first engagement member 2 includes two retention lugs 13, as shown in Fig. 11. As illustrated, along the direction from the distal end to proximal end, four first engagement members 2 (i.e., a first one, a second one, a third one, and a fourth one of the engagement members 2) are sequentially arranged at the proximal end 11 of the stent 1. The two retention lugs 13 in the most distal one of the first engagement members 2 (i.e., the first one of the first engagement members 2) are axially aligned those in the second one of the first engagement members 2, with those in the third one of the first engagement members 2 and the most proximal one of the first engagement members 2 (i.e., the fourth one of the first engagement members 2) being arranged in a similar manner. However, the retention lugs 13 in the latter two first engagement members 2 are axially staggered from those in the former two first engagement members 2. Such an arrangement with axial positions of the retention lugs 13 differing in the circumferential direction (i.e., being axially staggered) allows the stent 1 to have a reduced size when compressed and thus to be able to be delivered within a delivery sheath 5 have a smaller size.

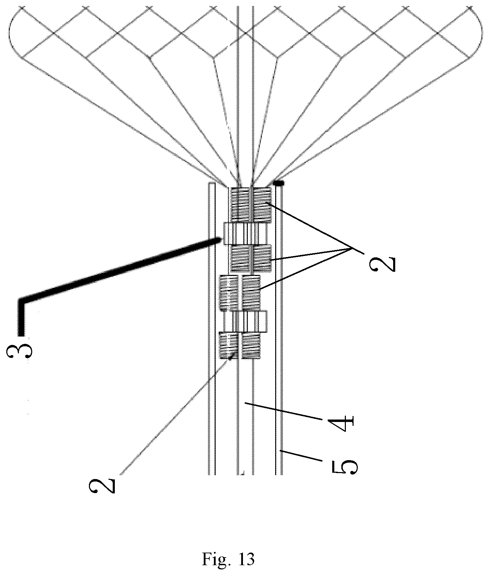

In this embodiment, as shown in Fig. 4, the delivery shaft 4 includes two second engagement members 3 for retaining engagement with the respective two first engagement member groups 14 and 15. As shown in Figs. 12 and 13, one of the second engagement members 3 is arranged between the proximal two of the first engagement members 2, and the other is arranged between the distal two of the first engagement members 2. In this way, the stent 1 and the delivery shaft 4 are limited relative to each other in terms of position, thus enabling stable, reliable engagement in addition to a reduced size.

### Embodiment 4

The stent and the delivery device according to this embodiment are similar in structure to those of the previous embodiments, and only its differences therefrom will be described below.

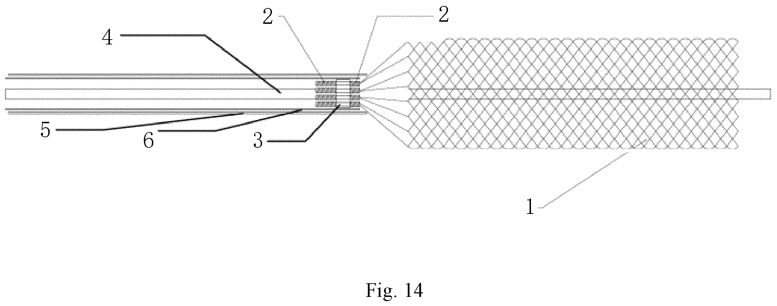

In this embodiment, as shown in Fig. 14, the delivery device further includes a release tube 6, which is movably disposed within the delivery sheath 5 over the delivery shaft 4 in order to enable entire release and entire retrieval of the stent 1. In this case, the release tube 6 makes up the confinement member.

Specifically, after the stent 1 is pushed out of the delivery sheath 5 at the target site, the proximal end 11 of the stent remains engaged with the delivery shaft 4. At this point, if it is found that the disengagement does not proceed very well (e.g., the stent is not entirely released or disengaged), the stent 1 can be entirely or partially withdrawn back into the delivery sheath 5 by moving back the delivery shaft 4. When it is determined that it would be appropriate to disengage the stent (e.g., correct release location has been attained), relative movement between the release tube 6 and the delivery shaft 4 may be caused. For example, the operating surgeon may push the delivery shaft 4 forward (distally), concurrently with the release tube 6 being maintained stationary. Alternatively, the release tube 6 may be withdrawn while the delivery shaft 4 is kept stationary. As a result, the proximal end 11 of the stent 1 is no longer radially confined by the release tube 6, thereby disengaging and separating the stent 1 from the delivery shaft 4. Compared to the prior art, the present disclosure allows the stent 1 (except the proximal end 11) to be entirely withdrawn after its entire release and subsequent disengagement of the proximal end 11 of the stent 1 from the delivery shaft 4 when satisfactory release location and condition have been confirmed. This allows increased release accuracy of the stent 1 and thus improved treatment outcomes.

More specifically, the release tube 6 so surrounds the second and first engagement members 3 and 2 that they remain in retaining engagement with each other. Moreover, if there is no relative movement between the delivery shaft 4 and the release tube 6, the stent 1 is always engaged with the delivery shaft 4. However, when the release tube 6 moves proximally relative to the delivery shaft 4, the proximal end 11 of the stent 1 and the second engagement member 3 will be no longer radially confined. Moreover, due to the self-expanding properties of the stent 1, the proximal end 11 of the stent 1 will radially flare away from the second engagement member 3 and thus disengage from the delivery shaft 4.

In this embodiment, the release tube 6 may extend distally up to the proximal end 11 of the stent 1 in order to confine the first engagement member(s) 2, and proximally beyond the proximal end of the delivery sheath 5 so as to be able to be manipulated by the surgeon. Further, the release tube 6 may be made of, but is not limited to, a macromolecular or metallic material.

Further, it is to be noted that, in the case of one second engagement member 3 being retained between two first engagement members 2, the two first engagement members 2 are axially spaced from each other, with their retention lugs 13 being axially either aligned or staggered. The staggered arrangement can result in a reduced size of the stent in the compressed configuration.

While how the stent and the delivery shaft are engaged with each other has been described in detail with reference to the above embodiments, it is a matter of course that the present disclosure is not limited to the engagement approaches disclosed. Those skilled in the art can devise other embodiments on the basis of the description of the foregoing embodiments.

## Claims

1. A vascular implant, for delivery to a target site by a delivery device comprising a delivery shaft (4) and a chamber, wherein the vascular implant comprises a proximal end (11) defining a first engagement structure configured for detachable retaining engagement with the delivery shaft (4), the first engagement structure and the delivery shaft (4) being able to be received in or removed from the chamber,
wherein when the first engagement structure and the delivery shaft (4) are received in the chamber, the first engagement structure is confined by the chamber and remains in retaining engagement with the delivery shaft (4), so that the vascular implant is axially locked to the delivery shaft (4), and
wherein when the first engagement structure is removed from the chamber, the first engagement structure is no longer confined by the chamber and is detachable from the delivery shaft (4), so that the vascular implant is axially unlocked from the delivery shaft (4);
wherein the first engagement structure comprises a first engagement member (2) configured to remain in retaining engagement with the delivery shaft (4) when confined by the chamber and to become detachable from the delivery shaft (4) when no longer confined by the chamber, wherein the first engagement member (2) defining a gap configured to:
disallow passage of the delivery shaft (4) therethrough when the first engagement structure and the delivery shaft (4) are received in the chamber to maintain the first engagement member (2) in retaining engagement with the delivery shaft (4); and
allow passage of the delivery shaft (4) therethrough when the first engagement structure is removed from the chamber to make the first engagement member (2) detachable from the delivery shaft (4);
wherein the first engagement member (2) comprises two or more retention lugs (13) which are spaced apart from one another along a circumferential direction of the vascular implant and define the gap therebetween;
wherein the vascular implant has a braided structure formed by braided filaments; the implant being **characterized in that** each of the retention lugs (13) is a coiled tube sleeved over and attached to filaments (S1, S2) bound together at the proximal end; and
wherein each of the retention lugs (13) is shaped as a coiled tube so as to be compressible along an axial direction of the vascular implant.

2. The vascular implant of claim 1, wherein at least one of the two or more retention lugs (13) is made of a radiopaque metallic material.

3. The vascular implant of claim 1, wherein the vascular implant is a self-expanding implant, in particular, a stent (1), an embolization coil or an aneurysm occluder.

## Patentansprüche

1. Vaskuläres Implantat zur Abgabe an eine Zielstelle durch eine Abgabevorrichtung, die einen Abgabeschaft (4) und eine Kammer umfasst, wobei das vaskuläre Implantat ein proximales Ende (11) umfasst, das eine erste Eingriffsstruktur definiert, die zum lösbaren Halteeingriff mit dem Abgabeschaft (4) ausgebildet ist, wobei die erste Eingriffsstruktur und der Abgabeschaft (4) in der Kammer aufnehmbar oder aus dieser entfernbar sind,
wobei, wenn die erste Eingriffsstruktur und der Abgabeschaft (4) in der Kammer aufgenommen sind, die erste Eingriffsstruktur durch die Kammer eingeschlossen ist und in Halteeingriff mit dem Abgabeschaft (4) bleibt, so dass das vaskuläre Implantat axial mit dem Abgabeschaft (4) verriegelt ist, und
wobei, wenn die erste Eingriffsstruktur aus der Kammer entfernt ist, die erste Eingriffsstruktur nicht länger durch die Kammer eingeschlossen ist und von dem Abgabeschaft (4) abnehmbar ist, so dass das vaskuläre Implantat axial von dem Abgabeschaft (4) entriegelt ist;
wobei die erste Eingriffsstruktur ein erstes Eingriffselement (2) umfasst, das ausgebildet ist, in Halteeingriff mit dem Abgabeschaft (4) zu bleiben, wenn diese durch die Kammer eingeschlossen ist, und von dem Abgabeschaft (4) lösbar ist, wenn diese nicht mehr durch die Kammer eingeschlossen ist, wobei das erste Eingriffselement (2) einen Spalt definiert, der ausgebildet ist:
dem Abgabeschaft (4) einen Durchgang dort hindurch zu verhindern, wenn die erste Eingriffsstruktur und der Abgabeschaft (4) in der Kammer aufgenommen sind, um das erste Eingriffselement (2) in Halteeingriff mit dem Abgabeschaft (4) zu halten; und
dem Abgabeschaft (4) den Durchgang dort hindurch zu ermöglichen, wenn die erste Eingriffsstruktur aus der Kammer entfernt ist, um das erste Eingriffselement (2) von dem Abgabeschaft (4) lösbar zu machen;
wobei das erste Eingriffselement (2) zwei oder mehr Haltelaschen (13) umfasst, die entlang einer Umfangsrichtung des vaskulären Implantats voneinander beabstandet sind und den Spalt dazwischen definieren;
wobei das vaskuläre Implantat eine geflochtene Struktur aufweist, die aus geflochtenen Filamenten gebildet wird; wobei das Implantat **dadurch gekennzeichnet ist, dass** jede der Haltelaschen (13) ein gewundenes Rohr ist, das über Filamente (S1, S2) gestülpt und an diesen befestigt ist, die an dem proximalen Ende miteinander verbunden sind; und
wobei jede der Haltelaschen (13) die Form eines gewundenen Rohrs hat, um entlang einer axialen Richtung des vaskulären Implantats komprimierbar zu sein.

2. Vaskuläres Implantat nach Anspruch 1, wobei mindestens eine der zwei oder mehr Haltelaschen (13) aus einem röntgendichten metallischen Material besteht.

3. Vaskuläres Implantat nach Anspruch 1, wobei das vaskuläre Implantat ein selbstexpandierendes Implantat, insbesondere ein Stent (1), eine Embolisationsspirale oder ein Aneurysma-Okkluder ist.

## Revendications

1. Implant vasculaire destiné à être délivré à un site cible par un dispositif de pose comprenant une tige de pose (4) et une chambre, dans lequel l'implant vasculaire comprend une extrémité proximale (11) définissant une première structure d'engagement configurée pour un engagement de retenue amovible avec la tige de pose (4), la première structure d'engagement et la tige de pose (4) pouvant être reçues dans la chambre ou retirées de celle-ci,
dans lequel lorsque la première structure d'engagement et l'arbre de distribution (4) sont reçus dans la chambre, la première structure d'engagement est confinée par la chambre et reste en engagement de retenue avec l'arbre de distribution (4), de sorte que l'implant vasculaire est verrouillé axialement sur l'arbre de distribution (4), et
dans lequel lorsque la première structure d'engagement est retirée de la chambre, la première structure d'engagement n'est plus confinée par la chambre et est détachable de la tige de distribution (4), de sorte que l'implant vasculaire est déverrouillé axialement de la tige de distribution (4) ;
dans laquelle la première structure d'engagement comprend un premier élément d'engagement (2) configuré pour rester en engagement de retenue avec l'arbre de distribution (4) lorsqu'il est confiné par la chambre et pour devenir détachable de l'arbre de distribution (4) lorsqu'il n'est plus confiné par la chambre, dans laquelle le premier élément d'engagement (2) définit un espace configuré pour :
interdire le passage de l'arbre de distribution (4) lorsque la première structure d'engagement et l'arbre de distribution (4) sont reçus dans la chambre pour maintenir le premier élément d'engagement (2) en engagement de maintien avec l'arbre de distribution (4) ; et
permettre le passage de l'arbre de distribution (4) à travers celui-ci lorsque la première structure d'engagement est retirée de la chambre pour rendre le premier élément d'engagement (2) détachable de l'arbre de distribution (4) ;
dans lequel le premier élément d'engagement (2) comprend deux ou plusieurs pattes de rétention (13) qui sont espacées les unes des autres le long d'une direction circonférentielle de l'implant vasculaire et définissent l'espace entre elles ;
dans lequel l'implant vasculaire présente une structure tressée formée de filaments tressés ; l'implant étant **caractérisé en ce que** chacune des pattes de rétention (13) est un tube spiralé gainé et fixé à des filaments (S1, S2) liés ensemble à l'extrémité proximale ; et
dans lequel chacune des pattes de rétention (13) est formée comme un tube enroulé de manière à être compressible le long d'une direction axiale de l'implant vasculaire.

2. Implant vasculaire selon la revendication 1, dans lequel au moins l'une des deux ou plusieurs pattes de rétention (13) est constituée d'un matériau métallique radio-opaque.

3. Implant vasculaire selon la revendication 1, dans lequel l'implant vasculaire est un implant auto-expansible, en particulier un stent (1), une bobine d'embolisation ou un obturateur d'anévrisme.
